# EUROPEAN PATENT APPLICATION

(11) **EP 3 219 208 A1**
(43) Date of publication of application: **20.09.2017**
(21) Application number: 15859034.9
(22) Date of filing: 13.11.2015
(51) Int. Cl.: A01N 55/02

(54) **ANTIMICROBIAL WATER DISPERSIBLE COMPOSITION COMPRISING ZINC PYRITHIONE**

(30) Priority: 14.11.2014 KR 20140159124
(71) Applicant: Kolon Life Science, Gwacheon-si, Gyeonggi-do 427-800 (KR)
(72) Inventor: LEE, Ji Hye, Seoul 135-520 (KR); JEON, Yong Suk, Incheon 404-320 (KR); CHO, Hee Won, Incheon 403-844 (KR); YANG, Ki Hoon, Yongin-si Gyeonggi-do 446-911 (KR); YOON, Hye Ji, Yongin-si Gyeonggi-do 446-771 (KR)
(74) Representative: Merkle, Gebhard
(86) International application number: PCT/KR2015/012194
(87) International publication number: WO 2016/076653

(57) **Abstract**

Disclosed is an antibiotic aqueous dispersion composition including as an active ingredient zinc pyrithione and, at least one subsidiary antiseptic selected from the group consisting of thiamine dilauryl sulphate (TDS), a salt and glyceryl monoalkyl ether. The composition has excellent stability and wide antibiotic or antiseptic spectra to bacteria and fungus.

## Description

### [Technical Field]

The present invention relates to an aqueous dispersion composition including zinc pyrithione and more particularly, to a zinc pyrithione aqueous dispersion composition which exhibits antibiotic activity to fungus and bacteria, while maintaining stability of the dispersion.

### [Background Art]

An antiseptic is an additive used for products such as food, medicines, paints, biological samples and wood to prevent decay caused by microorganism proliferation or undesired chemical changes, which is naturally derived or chemically synthesized. Microorganisms causing decay include molds and yeasts which belong to fungus, and bacteria and the like. Paraben-based antiseptics such as paraoxybenzoic ester and imidazolidinyl urea, phenoxyethanol and isothiazoline-based antiseptics have been used in order to eliminate microorganisms and thereby improve preservability of products. In addition, in accordance with market trends toward antiseptics safer to humans, diol-based antiseptic compositions are recently used and the majority thereof have limited applicability due to problems associated with skin irritation and miscibility.

Zinc pyrithione is a coordination complex of zinc, which is mainly used for hair cosmetic compositions and is reported to exhibit excellent inhibitory activity on scalp hyperkeratinization and superior antifungal efficacy. Particles of zinc pyrithione compounds are nearly insoluble in water. When the particles are used for shampoo and the like, dispersion becomes heterogeneous and interlayer separation occurs over time due to difference in density between the particles and water, and re-agglomeration of particles, and particles increase in size due to re-agglomeration. This phenomenon decreases surface areas of zinc pyrithione particles, deteriorates antibiotic activity as well as inhibits homogenous distribution of zinc pyrithione as a shampoo composition, thus having an adverse influence, when used for shampoo.

Meanwhile, an aqueous dispersion of zinc pyrithione is often reported to have problems associated with microorganism contamination during storage or transfer. Zinc pyrithione exhibits strong antibiotic activity to fungus, but it has poor antibiotic activity to bacteria (in particular, *P. aeruginosa).* Therefore, zinc pyrithione needs reinforced antibiotic activity to bacteria. In order to reinforce antibiotic activity to bacteria, isothiazolone-based antiseptics, which have been widely used as subsidiary antiseptics for zinc pyrithione aqueous dispersions, exhibit potent antibiotic activity to bacteria, but are recently limitedly used due to contact-type skin irritation.

Therefore, there is a need for development of zinc pyrithione aqueous dispersion compositions which maintain miscibility and stability in aqueous dispersions, and exhibit improved antibiotic activity to bacteria.

### [Prior Art]

### [Patent Document]

(Patent Document 1) US Patent No. 5854266 (issued on January 29, 1998)
(Patent Document 2) EP Patent No. 1527684 (issued on February 27, 2013)
(Patent Document 3) US Patent No. 5716628 (issued on February 10, 1998)
(Patent Document 4) JP Patent Publication Patent No. 2013-189404 (published on September 26, 2013)
(Patent Document 5) US Patent Publication No. 2010-0137272 (published on June 3, 2010)

### [Disclosure]

### [Technical Problem]

Therefore, the present invention has been made in view of the above problems, and it is one object of the present invention to provide a composition which maintains stability of a zinc pyrithione aqueous dispersion and has wide antibiotic or antiseptic spectra to bacteria and fungus.

### [Technical Solution]

In accordance with the present invention, the above and other objects can be accomplished by the provision of an antibiotic aqueous dispersion composition including as an active ingredient zinc pyrithione and, at least one subsidiary antiseptic selected from the group consisting of thiamine dilauryl sulphate (TDS), a salt and glyceryl monoalkyl ether.

The subsidiary antiseptic may be a combination of thiamine dilauryl sulphate with any one selected from the group consisting of a salt and glyceryl monoalkyl ether and a mixture thereof.

The salt may include at least one organic acid salt selected from the group consisting of sulfite, metabisulfite and salicylate.

The glyceryl monoalkyl ether may include at least one selected from the group consisting of ethylhexylglycerin and caprylyl glyceryl ether.

The subsidiary antiseptic may be a combination of three types of subsidiary antiseptics including thiamine dilauryl sulphate, a salt and glyceryl monoalkyl ether. In this case, the subsidiary antiseptic may include thiamine dilauryl sulphate, at least one organic acid salt selected from the group consisting of sulfite, metabisulfite and salicylate, and at least one glyceryl monoalkyl ether selected from the group consisting of ethylhexyl glycerin and caprylyl glyceryl ether.

In addition, the subsidiary antiseptic may include 100 parts by weight of the thiamine dilauryl sulphate, 20 to 200 parts by weight of the organic acid salt, and 20 to 100 parts by weight of the glyceryl monoalkyl ether.

The thiamine dilauryl sulphate may be present in an amount of 0.01 to 0.1% by weight, based on the total weight of the composition.

The salt may be present in an amount of 0.005 to 0.2% by weight, based on the total weight of the composition.

The glyceryl monoalkyl ether may be present in an amount of 0.005 to 0.1% by weight, based on the total weight of the composition.

The antibiotic aqueous dispersion composition may have antibiotic activity to both fungus and bacteria.

The antibiotic aqueous dispersion composition may have antibiotic activity to all of *E. coli, Staphylococcus aureus, Pseudomonas aeruginosa, Candida albicans* and *Aspergillus niger.*

### [Effects of the Invention]

The antibiotic aqueous dispersion composition according to the present invention may have a wide range of antibiotic or antiseptic activity to microorganisms including fungus and bacteria. In addition, the antibiotic aqueous dispersion composition has a wide range of antibiotic or antiseptic activity and at the same time, maintains stability of zinc pyrithione aqueous dispersions and exhibits more excellent antibiotic activity when used in combination.

In particular, the antibiotic aqueous dispersion composition according to the present invention can improve preservability of zinc pyrithione aqueous dispersions vulnerable to bacteria (in particular, *P. aeruginosa),* thereby extending a use range of the zinc pyrithione aqueous dispersions.

### [Best Mode]

Hereinafter, the present invention will be described in detail.

The inventors of the present invention confirmed that conditions for maintaining the stability of zinc pyrithione aqueous dispersions are different from conditions for maximizing activity of subsidiary antiseptics, and discovered antiseptic mix compositions with excellent antibiotic activity to bacteria as well as improved miscibility and stability of products, as an alternative to isothiazolone-based antiseptics that involve usage restrictions due to skin irritation, thereby completing the present invention.

In one aspect, the present invention is directed to an antibiotic aqueous dispersion composition including as an active ingredient zinc pyrithione and, at least one subsidiary antiseptic selected from the group consisting of thiamine dilauryl sulphate (TDS), a salt and glyceryl monoalkyl ether.

The antibiotic composition has the same meaning as an antibiotic that generically refers to an antimicrobial agent and may have the same meaning as an antimycotic, germicide, antiseptic, preservative or fungicide.

The expression "including as an active ingredient" means that the corresponding ingredient is present in a sufficient amount to exert desired efficacy or activity. The antibiotic aqueous dispersion composition may include the active ingredient in an amount of 0.001 to 99.99% by weight, preferably 0.01 to 99% by weight, more preferably 0.1 to 10% by weight, with respect to the total weight of the composition, but the content of the active ingredient can be suitably controlled depending on the use method and purpose of the antibiotic aqueous dispersion composition.

Zinc pyrithione is a coordination salt of zinc, which is represented by the following Formula 1. Zinc pyrithione is widely used as a drug for skin diseases such as seborrheic dermatitis of the scalp and an ingredient for antifouling paints due to antibiotic activity thereof.

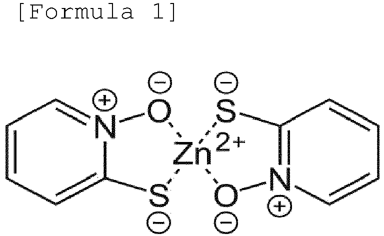

Zinc pyrithione may be present in an amount of 20 to 80% by weight, preferably 25 to 65% by weight, based on the total weight of the composition. When the content of the zinc pyrithione is less than 20% by weight, stability of final shampoo or rinse formulations may be deteriorated and there is no benefit in terms of costs, because more thickening agents or subsidiary stabilizers than conventional cases should be used in order to produce aqueous dispersion compositions with excellent storage stability. When the content of zinc pyrithione is higher than 80% by weight, it is difficult to use and uniformly feed zinc pyrithione in the process of antifouling paints and/or paints for exterior use upon manufacture of final products such as cosmetics or personal care products in the form of a high-viscosity paste.

The subsidiary antiseptic refers to an antibiotic substance which is added to the composition to enhance antibiotic activity of zinc pyrithione and may include one or more substances selected from the group consisting of thiamine dilauryl sulphate, a salt and glyceryl monoalkyl ether. The subsidiary antiseptic is added to products requiring an antiseptic effect so that it can exert effects of improving storage stability of products and preventing microorganism contamination. In addition, the subsidiary antiseptic can impart a wide range of antibiotic and antiseptic spectra which are difficult to obtain with zinc pyrithione alone and maintain stability of zinc pyrithione aqueous dispersions.

The subsidiary antiseptic may be a two-component combination subsidiary antiseptic which includes thiamine dilauryl sulphate as an essential ingredient and is obtained by combining the same with a salt or glyceryl monoalkyl ether. In addition, the subsidiary antiseptic may be a three-component combination subsidiary antiseptic which includes thiamine dilauryl sulphate, a salt and glyceryl monoalkyl ether.

Thiamine dilauryl sulphate is a derivative of vitamin B1 (thiamine) which exhibits antibiotic activity to bacteria and/or fungus, structural formula of which is represented by the following Formula 2.

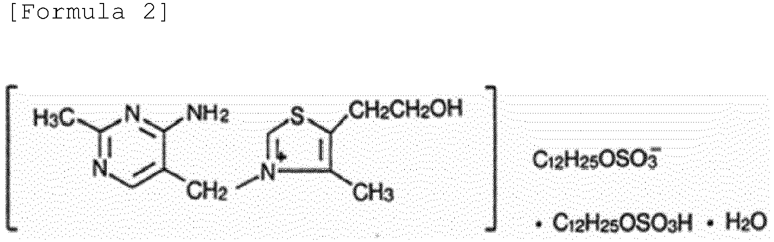

The thiamine dilauryl sulphate is a safe substance which is used as a preservative for food, which is highly insoluble in water, but may be soluble in water under specific conditions, *i.e.,* in the presence of a surfactant. The thiamine dilauryl sulphate can exert synergistic antibiotic activity when used in combination with zinc pyrithione and can improve miscibility and/or preservability of zinc pyrithione aqueous dispersions vulnerable to bacteria (in particular, *P. aeruginosa).*

The thiamine dilauryl sulphate is preferably present in an amount of 0.01 to 0.10% by weight, preferably 0.03 to 0.05% by weight in the composition. When the content of the thiamine dilauryl sulphate is less than 0.01% by weight, antibiotic synergy of zinc pyrithione is insufficient and when the content exceeds 0.10% by weight, it may be precipitated due to low solubility and thus have stability problems.

The salt refers to a salt with antibiotic activity which is used as a preservative for food and is preferably a salt of organic acid. More preferably, the salt may include one or more organic acids selected from the group consisting of benzoate, sorbate, sulfite, metabisulfite and salicylate, but it is not limited thereto. The salt may include a variety of salts or derivatives thereof which have antibiotic activity as subsidiary antiseptics and miscibility with zinc pyrithione. The salt is preferably a sodium salt.

The salt is preferably present in an amount of 0.005 to 0.20% by weight, preferably 0.01 to 0.10% by weight, in the composition. When the content of the salt is less than 0.005% by weight, antibiotic synergy with zinc pyrithione is insufficient and, when the content exceeds 0.20% by weight, deterioration in viscosity of the zinc pyrithione aqueous dispersion composition and stability-associated problems may occur due to low pH.

Glyceryl monoalkyl ether is a substance having a structure in which one aliphatic chain is ether-bonded to glycerin, which has antibiotic activity to bacteria and/or fungus. The glyceryl monoalkyl ether is preferably selected from the group consisting of ethylhexylglycerin, caprylyl glyceryl ether and mixtures thereof, but the present invention is not limited thereto. The glyceryl monoalkyl ether may include a variety of glyceryl monoalkyl ethers or derivatives thereof which have antibiotic activity as subsidiary antiseptics and miscibility with zinc pyrithione.

The glyceryl monoalkyl ether may be preferably present in an amount of 0.005 to 0.1% by weight, preferably 0.01 to 0.05% by weight in the composition. When the content of the glyceryl monoalkyl ether is less than 0.005% by weight, antibiotic synergy with zinc pyrithione is insufficient, and when the content exceeds 0.10% by weight, stability problem of the zinc pyrithione aqueous dispersion composition may occur.

The subsidiary antiseptic including thiamine dilauryl sulphate, a salt and glyceryl monoalkyl ether may be used as a single antiseptic. However, in this case, the subsidiary antiseptic may exhibit slight antibiotic activity to specific microorganisms. Therefore, in the present invention, the subsidiary antiseptic may be used in combination in order to exert excellent activity, miscibility with the zinc pyrithione aqueous dispersion, and stable aqueous dispersibility, as compared to conventional antiseptics. Preferably, the antibiotic aqueous dispersion composition may include two or three among the three subsidiary antiseptics. When the antibiotic aqueous dispersion composition includes all three subsidiary antiseptics, preferably, the thiamine dilauryl sulphate may be present in an amount of 0.01 to 0.10% by weight, the salt may be present in an amount of 0.005 to 0.20% by weight and the glyceryl monoalkyl ether may be present in an amount of 0.005 to 0.10% by weight in the antibiotic aqueous dispersion composition.

When the antibiotic aqueous dispersion composition includes all three subsidiary antiseptics, the subsidiary antiseptic may include: one or more organic acid salts selected from the group consisting of thiamine dilauryl sulphate, sulfite, metabisulfite and salicylate; and one or more glyceryl monoalkyl ether selected from the group consisting of ethylhexylglycerin and caprylyl glyceryl ether.

In addition, the subsidiary antiseptic may include 100 parts by weight of the thiamine dilauryl sulphate, 20 to 200 parts by weight of the organic acid salt, and 20 to 100 parts by weight of the glyceryl monoalkyl ether.

The antibiotic aqueous dispersion composition can exhibit antibiotic activity to both fungus and bacteria based on synergistic antibiotic activity of zinc pyrithione with the subsidiary antiseptic, and overcome vulnerability of the zinc pyrithione aqueous dispersion to bacteria (in particular, *P. aeruginosa)* and impart a wide range of antibiotic and antiseptic spectra. Based on this point, the antibiotic aqueous dispersion composition preferably exhibits antibiotic activity to all of *E. coli, S. aureus, P. aeruginosa, C. albicans* and *A. niger.*

The antibiotic aqueous dispersion composition can exhibit excellent miscibility and stability in the aqueous dispersion via a suitable combination of zinc pyrithione and the subsidiary antiseptic. Preferably, the antibiotic aqueous dispersion composition may have dispersion stability (Turbiscan stability index, measured for 2 weeks at 60°C) of 4.5 or less.

Meanwhile, when a part includes one constituent component, it may further include other constituent components without exclusion thereof, unless mentioned herein otherwise, and those skilled in the art will understand that the antibiotic aqueous dispersion composition may include a variety of additional ingredients depending on the purpose or formulation used.

### [Mode for the Invention]

Hereinafter, embodiments of the present invention will be described in more detail such that a person having ordinary knowledge in the field to which the present invention pertains can easily implement the embodiments. However, the embodiments of the present invention can be implemented in various forms and should not be construed as being limited to the embodiments described herein.

### Comparative Example: Measurement of antibiotic activity and stability of conventional antiseptics

In order to confirm antibiotic activity and stability of the aqueous dispersion composition according to the present invention, first, antibiotic activity and stability of conventional antiseptics were measured.

First, antibiotic activity was evaluated using the following method.

Respective samples were inoculated with three kinds of bacteria and two kinds of fungus and cultured for 7 days in order to measure antibiotic activity. On the first, second, third and seventh days, sampling was conducted, the sample was spread on a solid medium and antibiotic activity was checked. Strains used to measure antibiotic activity were *Escherichia coli, Staphylococcus aureus, Pseudomonas aeruginosa* for bacteria, and *Candida albicans* and *Aspergillus niger* for fungus. Regarding the media used respectively for the strains, TSA (tryptic soy agar) and NB (nutrient broth) were used for the three types of bacteria, and SDA (Sabouraud dextrose agar) and YM (yeast extract and malt extract) were used for *Candida albicans* (*C. albicans*)*,* and SDA (Sabouraud dextrose agar) and MD (malt extract and dextrose) were used for *Aspergillus niger* (*A. niger*).

Antibiotic activity was evaluated based on the day on which all strains completely died, rather than lived and grew in the sample spread on the solid medium. First day death was considered to be excellent, second day death was considered to be good, third day death was considered to be medium, seventh day death was considered to be bad and no death until the seventh day was considered to be extremely bad.

A more detailed test method is as follows. A 50 ml sterilized container did not include zinc pyrithione and included all other ingredients such as a thickening agent and a dispersant. 50 g of an aqueous dispersion supplemented with a suitable concentration of antibiotic agent was added to the container to prepare a sample. In consideration of the test method, in order to test zinc pyrithione having antibiotic activity, antibiotic activity of zinc pyrithione was preferably removed using a neutralization agent, but water was inevitably used to adjust a predetermined volume without adding zinc pyrithione due to absence of a suitable neutralization agent.

The prepared sample was inoculated with 1.0 x 10⁶ cells/ml *of E. coli, S. aureus* and *P. aeruginosa,* and with 1.0 x 10⁵ cells/ml *of C. albicans* and *A. niger.* Then, *E*. *coli, S. aureus* and *P. aeruginosa* were cultured at 37°C, while *C*. *albicans* and *A. niger* were cultured at 30°C. At the point of times of 1, 2, 3 and 7 days after culture, 100 µl of the sample was obtained and spread on a solid medium and *E. coli, S. aureus* and *P. aeruginosa* were then cultured at 37°C for 1 day for, and *C*. *albicans* and *A. niger* were cultured at 30°C for 2 days. Then, whether the strains were alive or dead was checked. The respective antiseptics were used with reference to optimal pH, dosage and MIC recommended by the company of manufacture, wherein the test pH conditions were pH 5.5 to 9.0 at which zinc pyrithione was stable.

In addition, the stability of the zinc pyrithione aqueous dispersion was measured under the following conditions.

First, a zinc pyrithione aqueous dispersion including an antiseptic which was identified to have antiseptic activity was prepared and then stored at room temperature (R.T.), at a temperature ranging from 10°C to 20°C, or 60°C, and variation in physical properties related to stability and dispersibility were checked.

Variation in physical properties was determined depending on whether or not assay, viscosity, pH and particle size were changed, and variation in dispersibility was determined by checking layer separation degree using centrifugation and measuring dispersion stability using Turbiscan for detecting particle size variation and phase separation.

Stability was evaluated by checking a layer separation degree (production of supernatant) using centrifugation by the naked eye and was divided into grades based on the proportion of the height of the layer-separated supernatant with respect to the total height of the sample. With respect to the grades, layer separation degree (production of supernatant) of less than 5% was regarded as "high", layer separation degree of 10% or less was regarded as "medium-high", layer separation degree of 15% or less was regarded as "medium", layer separation degree of 20% or less was regarded as "medium-low" and layer separation degree of higher than 20% was regarded as "low".

Meanwhile, from the result of determination of stability via separate testing of the zinc pyrithione aqueous dispersion composition using the Turbiscan, it could seen that the result obtained by storing at 60°C for 2 weeks was similar to the result obtained by storing at room temperature for 3 months. Therefore, stability of the zinc pyrithione aqueous dispersion was compared based on stability index (Turbiscan stability index) associated with the result obtained by storing the test subject substances at 60°C for 2 weeks.

The test subject substances, that is, conventionally used antiseptics were MIT (methylisothiazolinone), CMIT (chloromethylisothiazolinone), DMDMH (dimethylol dimethylhydantoin) and a combination thereof.

First, results of antibiotic activity and stability measured for the conventionally used antiseptics are shown in the following Table 1. In the following Table 1, stability represents a layer separation degree via centrifugation observed with the naked eye.

**TABLE 1**

| Challenge test | MIT | MIT+CMIT | DMDMH | DMDMH+MI T |
|---|---|---|---|---|
| *E.coli* | Excellent | Excellent | Excellent | Excellent |
| *S.Aureus* | Excellent | Excellent | Excellent | Excellent |
| *P. aeruginosa* | Excellent | Excellent | Excellent | Excellent |
| *C. albicans* | Excellent | Excellent | Excellent | Excellent |
| *A.niger* | Excellent | Excellent | Excellent | Excellent |
| Stability of zinc pyrithione aqueous dispersion | High | High | High | High |

In addition, the stability of conventionally used antiseptics was measured as Turbiscan-related stability and the result thus obtained is shown as a stability index in the following Table 2. As the number of the stability index shown in the following Table 2 decreases, stability increases and zero (0) means no variation.

**TABLE 2**

| 60°C X 2 weeks | MIT (NP_1101-1) | MIT+CMIT (MIT_1201-1) | DMDMH (SP_1101-3) | DMDMH+MIT (SP_1211-3) |
|---|---|---|---|---|
| Stability Index | 3.9 | 4.5 | 3.9 | 4.8 |

### Example 1: Confirmation of stability when using subsidiary antiseptic alone

When one kind of subsidiary antiseptic alone was added to the zinc pyrithione aqueous dispersion, stability of the aqueous dispersion was confirmed by observation of the layer separation degree by the naked eye. The results are shown in the following Tables 3 to 5.

**TABLE 3**

| Items | Salts | | | | | |
|---|---|---|---|---|---|---|
| | Thiamine dilauryl sulfate | Sodium sulfite | Sodium metabisulfite | Sodium salicylate | Sodium benzoate | Potassium sorbate |
| Stability of zinc pyrithione aqueous dispersion | High | High | High | Medium-high | Medium-high | Medium-high |

**TABLE 4**

| Items | Glyceryl monoalkylehter | | Diol | |
|---|---|---|---|---|
| | Ethylhexyl glycerin | Caprylyl glyceryl ether | 1,2-hexanediol | 1,2-octanediol |
| Stability of zinc pyrithione aqueous dispersion | Medium-high | Medium-high | Medium | Medium |

**TABLE 5**

| Items | Alcohol | | Acid | |
|---|---|---|---|---|
| | Benzyl alcohol | Ethanol | Citric acid | Sorbic acid |
| Stability of zinc pyrithione aqueous dispersion | Medium-low | Medium-low | Low | Low |

As can be seen from Tables 3 to 5, stability of the zinc pyrithione aqueous dispersion using a subsidiary antiseptic alone was confirmed and, as a result, the salt subsidiary antiseptic was determined to be best and exhibited similar effects to conventionally used antiseptics, while acidic antiseptics were determined to have bad results.

Accordingly, the salt (stability: high) and glyceryl monoalkyl ether (stability: medium-high) were used as subsidiary antiseptics that could be used in combination with zinc pyrithione in consideration of stability of the zinc pyrithione aqueous dispersion.

### Example 2: Confirmation of antibiotic activity when using subsidiary antiseptic alone

When one kind of subsidiary antiseptic alone was added to the zinc pyrithione aqueous dispersion, antibiotic activity of the aqueous dispersion was tested. The results are shown in the following Tables 6 and 7.

**TABLE 6**

| Challenge test | ①Thiamine dilauryl sulfate | ②Sodium sulfite | ③Sodium metabi sulfite | ④Sodium salicylate | ⑤ Sodium benzoate | ⑥ Potassium sorbate |
|---|---|---|---|---|---|---|
| *E. coli* | Good | Good | Good | Good | Good | Good |
| *S. aureus* | Excellent | Good | Good | Good | Good | Good |
| *P*. *aeruginosa* | Good | Good | Good | Good | Good | Good |
| *C. albicans* | Good | Extremely bad | Extremely bad | Extremely bad | Extremely bad | Extremely bad |
| *A. niger* | Bad | Good | Good | Good | Good | Good |

**TABLE 7**

| Challenge test | ⑦ Ethylhexyl glycerin | ⑧ Caprylyl glyceryl ether |
|---|---|---|
| *E. coli* | Good | Good |
| *S. aureus* | Good | Good |
| *P. aeruginosa* | Extremely bad | Extremely bad |
| *C. albicans* | Good | Good |
| *A. niger* | Medium | Medium |

As a result of confirmation of antibiotic activity of substances that belong to subsidiary antiseptics having good stability of the zinc pyrithione aqueous dispersion in Example 1, thiamine dilauryl sulphate was vulnerable to only *A. niger,* while other salts were vulnerable to *C. albicans.* In addition, glyceryl monoalkyl ether was determined to be vulnerable to *P. aeruginosa.*

As such, when only one kind of subsidiary antiseptic was used, antibiotic activity to all of a variety of bacteria could not be obtained.

### Example 3: Confirmation of antibiotic activity and stability when using a combination of two kinds of subsidiary antiseptics

When two kinds of subsidiary antiseptics were added in combination to the zinc pyrithione aqueous dispersion, antibiotic activity and stability of the aqueous dispersion were tested. The results are shown in the following Tables 8 to 10.

**TABLE 8**

| Challenge test | ① Thiamine dilauryl sulfate + ② Sodium sulfite | ① Thiamine dilauryl sulfate + ③ Sodium metabisulfite | ① Thiamine dilauryl sulfate + ④ Sodium salicylate |
|---|---|---|---|
| *E. coli* | Good | Good | Good |
| *S. aureus* | Excellent | Excellent | Excellent |
| *P. aeruginosa* | Excellent | Excellent | Excellent |
| *C. albicans* | Good | Good | Good |
| *A. niger* | Good | Good | Good |
| Stability of zinc pyrithione aqueous dispersion | Medium-high | Medium-high | Medium-high |
| Stability index at 60°C X 2 weeks | 3.0 | 3.4 | 4.2 |

**TABLE 9**

| Challenge test | ①Thiamine dilauryl sulfate + ⑤ sodium benzoate | ①Thiamine dilauryl sulfate + ⑥ potassium sorbate |
|---|---|---|
| *E. coli* | Good | Good |
| *S. aureus* | Excellent | Excellent |
| *P. aeruginosa* | Excellent | Excellent |
| *C. albicans* | Good | Good |
| *A. niger* | Good | Good |
| Stability of zinc pyrithione aqueous dispersion | Medium-high | Medium-high |
| Stability index at 60°C X 2 weeks | 4.5 | 4.3 |

**TABLE 10**

| Challenge test | ①Thiamine dilauryl sulfate + ⑦Ethylhexyl glycerin | ①Thiamine dilauryl sulfate + ⑧Caprylyl glyceryl ether |
|---|---|---|
| *E. coli* | Good | Good |
| *S. aureus* | Excellent | Excellent |
| *P. aeruginosa* | Good | Good |
| *C. albicans* | Good | Good |
| *A. niger* | Good | Good |
| Stability of zinc pyrithione aqueous dispersion | Medium-high | Medium-high |
| Stability index at 60°C X 2 weeks | 4.0 | 4.3 |

Compared with the result of confirmation of antibiotic activity of a single antiseptic in Example 2, antibiotic activity of two kinds of antiseptic combinations, i.e., a combination of thiamine dilauryl sulphate and a salt, specifically, sulfite (sodium sulfite) or salicylate (sodium salicylate) in Example 3 was checked. As a result, the antiseptic combination exhibited synergistic antibiotic activity to *P. aeruginosa* and *C. albicans.*

In addition, antibiotic activity of the two-kind combination antiseptic obtained by mixing thiamine dilauryl sulphate and glyceryl monoalkyl ether, specifically, ethylhexylglycerin or caprylyl glyceryl ether was confirmed. As a result, the combination antiseptic was determined to exhibit synergistically antibiotic activity to *P. aeruginosa* and *A.niger.*

In addition, taking into consideration the result of confirming the stability of the zinc pyrithione aqueous dispersion by the naked eye and stability index measured using a Turbiscan device, the stability was determined to have no problems, as compared to products utilizing conventionally used antiseptics of Comparative Example.

### Example 4: Confirmation of antibiotic activity and stability when using a combination of three kinds of subsidiary antiseptics

When three kinds of subsidiary antiseptic were added in combination to the zinc pyrithione aqueous dispersion, antibiotic activity and stability of the aqueous dispersion were tested. The results are shown in the following Tables 11 and 12.

**TABLE 11**

| Challenge test | ① Thiamine dilauryl sulphate + ② Sodium sulfite +⑦ Ethylhexyl glycerin | ① Thiamine dilauryl sulphate+ ③ Sodium metabisulfite + ⑦ Ethylhexyl glycerin | ① Thiamine dilauryl sulphate + ④ Sodium salicylate + ⑦ Ethylhexyl glycerin | ① Thiamine dilauryl sulfate + ⑤ sodium benzoate+ ⑦ Ethylhexyl glycerin | ① Thiamine dilauryl sulfate + ⑥ Potassium sorbate+ ⑦ Ethylhexyl glycerin |
|---|---|---|---|---|---|
| *E. coli* | Excellent | Excellent | Excellent | Excellent | Excellent |
| *S. aureus* | Excellent | Excellent | Excellent | Excellent | Excellent |
| *P. aeruginosa* | Excellent | Excellent | Excellent | Excellent | Excellent |
| *C. albicans* | Excellent | Excellent | Excellent | Excellent | Excellent |
| *A. niger* | Excellent | Excellent | Excellent | Excellent | Excellent |
| Stability of zinc pyrithione aqueous dispersion | Medium-high | Medium-high | Medium-high | Medium-high | Medium-high |
| Stability index at 60°C X 2 weeks | 3.3 | 4.1 | 4.0 | 4.5 | 4.3 |

**TABLE 12**

| Challenge test | ① Thiamine dilauryl sulphate + ② Sodium sulfite + ⑧ Caprylyl glyceryl ether | ① Thiamine dilauryl sulphate + ③ Sodium metabisulfite + ⑧ Caprylyl glyceryl ether | ① Thiamine dilauryl sulphate + ④ Sodium salicylate + ⑧ Caprylyl glyceryl ether | ① Thiamine dilauryl sulfate + ⑤ Sodium benzoate+ ⑧ Caprylyl glyceryl ether | ① Thiamine dilauryl sulfate + ⑥ Potassium sorbate+ ⑧ Caprylyl glyceryl ether |
|---|---|---|---|---|---|
| *E. coli* | Excellent | Excellent | Excellent | Excellent | Excellent |
| *S. aureus* | Excellent | Excellent | Excellent | Excellent | Excellent |
| *P. aeruginosa* | Excellent | Excellent | Excellent | Excellent | Excellent |
| *C*. *albicans* | Excellent | Excellent | Excellent | Excellent | Excellent |
| *A.niger* | Excellent | Excellent | Excellent | Excellent | Excellent |
| Stability of zinc pyrithione aqueous dispersion | Medium-high | Medium-high | Medium-high | Medium-high | Medium-high |
| Stability index at 60°C X 2 weeks | 4.2 | 3.8 | 4.5 | 4.3 | 4.5 |

Compared with antibiotic activity of the combination antiseptic of thiamine dilauryl sulphate and a salt in Example 3, antibiotic activity of the three-kind combination subsidiary antiseptic of diamine dilauryl sulfate, a salt and glyceryl monoalkyl ether in Example 4 was confirmed. As a result, the three-kind combination subsidiary antiseptic was determined to exhibit synergistic antibiotic activity to *E. coli, C. albicans* and *A. niger.* Furthermore, the combination subsidiary antiseptic secured antibiotic activity (excellent) to five harmful strains described in the USP (the U.S. Pharmacopeial Convention).

In addition, it could be seen from stability of the zinc pyrithione aqueous dispersion observed by the naked eye and stability index measured using a Turbiscan device, that the combination subsidiary antiseptic exhibited better stability than the conventionally used antiseptic of Comparative Example 1.

The description of the present invention given above is provided for illustration of the present invention and those skilled having ordinary knowledge in the field to which the present invention pertains can understand that the present invention can be easily modified into other specific forms without changing technical concepts or indispensable features of the present invention. Therefore, the embodiments mentioned above are provided only for illustration and should not be construed as limiting the scope of the present invention. For example, respective constituent elements described as a singular form may be implemented separately. Similarly, respective constituent elements described as being separate may be implemented in an integrated form.

The scope of the present invention is defined by the following claims rather than the detailed description of the Invention and it should be interpreted that all modifications or alterations derived from the scope of the claims and equivalents thereto are incorporated into the range of the present invention.

### [Industrial Applicability]

The present invention relates to an antibiotic aqueous dispersion composition including zinc pyrithione. The antibiotic aqueous dispersion composition has a wide range of antibiotic or antiseptic activity to microorganisms including fungus and bacteria, maintains stability of the zinc pyrithione aqueous dispersion and exhibits more excellent antibiotic activity when used in combination. In particular, the antibiotic aqueous dispersion composition according to the present invention can improve preservability of zinc pyrithione aqueous dispersions vulnerable to bacteria (in particular, *P. aeruginosa),* thereby extending a use range of the zinc pyrithione aqueous dispersions.

## Claims

1. An antibiotic aqueous dispersion composition comprising as an active ingredient:
zinc pyrithione; and
at least one subsidiary antiseptic selected from the group consisting of thiamine dilauryl sulphate (TDS), a salt and glyceryl monoalkyl ether.

2. The antibiotic aqueous dispersion composition according to claim 1, wherein the subsidiary antiseptic is a combination of thiamine dilauryl sulphate with any one selected from the group consisting of a salt and glyceryl monoalkyl ether and a mixture thereof.

3. The antibiotic aqueous dispersion composition according to claim 1, wherein the salt is at least one organic acid salt selected from the group consisting of sulfite, metabisulfite and salicylate.

4. The antibiotic aqueous dispersion composition according to claim 1, wherein the glyceryl monoalkyl ether comprises at least one selected from the group consisting of ethylhexylglycerin and caprylyl glyceryl ether.

5. The antibiotic aqueous dispersion composition according to claim 1, wherein the subsidiary antiseptic comprises:
thiamine dilauryl sulphate;
at least one organic acid salt selected from the group consisting of sulfite, metabisulfite and salicylate; and
at least one glyceryl monoalkyl ether selected from the group consisting of ethylhexyl glycerin and caprylyl glyceryl ether.

6. The antibiotic aqueous dispersion composition according to claim 5, wherein the subsidiary antiseptic comprises 100 parts by weight of the thiamine dilauryl sulphate, 20 to 200 parts by weight of the organic acid salt, and 20 to 100 parts by weight of the glyceryl monoalkyl ether.

7. The antibiotic aqueous dispersion composition according to claim 1, wherein the thiamine dilauryl sulphate is present in an amount of 0.01 to 0.10% by weight, based on the total weight of the composition.

8. The antibiotic aqueous dispersion composition according to claim 1, wherein the salt is present in an amount of 0.005 to 0.20% by weight, based on the total weight of the composition.

9. The antibiotic aqueous dispersion composition according to claim 1, wherein the glyceryl monoalkyl ether is present in an amount of 0.005 to 0.10% by weight, based on the total weight of the composition.

10. The antibiotic aqueous dispersion composition according to claim 1, wherein the antibiotic aqueous dispersion composition has antibiotic activity to both fungus and bacteria.

11. The antibiotic aqueous dispersion composition according to claim 10, wherein the antibiotic aqueous dispersion composition has antibiotic activity to all of *E. coli, Staphylococcus aureus, Pseudomonas aeruginosa, Candida albicans* and *Aspergillus niger.*
